# FASCICULE DE BREVET EUROPEEN

(11) **EP 1 311 708 B1**
(45) Date de publication et mention de la délivrance du brevet: **03.09.2008**
(21) Numéro de dépôt: 01965340.1
(22) Date de dépôt: 22.08.2001
(51) Int. Cl.: C12Q 1/68, C12N 15/63, C12N 15/85, C12N 5/10, G01N 33/566, A61P 29/00, A61K 31/00

(54) **PROCEDE D'IDENTIFICATION DE SUBSTANCES UTILES POUR LE TRAITEMENT DE L'INFLAMMATION METTANT EN OEUVRE L'ELEMENT DE REPONSE AU RECEPTEUR ROR DU GENE IKAPPABALPHA**
VERFAHREN ZUR IDENTIFIZIERUNG VON SUBSTANZEN ZUR BEHANDLUNG VON ENTZÜNDUNGEN DURCH VERBRAUCH VOM ROR-ANTWORT ELEMENT DES IKAPPABALPHA GENS
METHOD FOR IDENTIFYING SUBSTANCES USEFUL FOR TREATING INFLAMMATION USING THE RESPONSE ELEMENT TO THE IKAPPABAPLHA ROR RECEPTOR

(30) Priorité: 23.08.2000 FR 0010857
(43) Date de publication de la demande: 21.05.2003
(73) Titulaire: Genfit, 59120 Loos (FR)
(72) Inventeur: STAELS, Bart, B-7850 Petit Enghien (BE); DELERIVE, Philippe, F-92300 LEVALLOIS PERRET (FR)
(74) Mandataire: Becker, Philippe
(86) Numéro de dépôt international: PCT/FR2001/002652
(87) Numéro de publication internationale: WO 2002/016638

(56) Documents cités:
- EP-A- 0 779 361
- WO-A-99/17778
- WO-A-99/65449
- FR-A- 2 776 388
- US-A- 5 747 661
- US-A- 5 846 714
- US-A- 5 939 285
- US-A- 5 958 683
- ITO C ET AL: "Three NF-kappaB sites in the IkappaB-alpha promoter are required for induction of gene expression by TNFalpha" NUCLEIC ACIDS RESEARCH, vol. 22, no. 18, septembre 1994 (1994-09), pages 3787-92, XP002185746
- DELERIVE P ET AL: "Induction of IkappaBalpha expression as a mechanism contributing to the anti-inflammatory activities of peroxisome proliferator-activated receptor-alpha activators" THE JOURNAL OF BIOLOGICAL CHEMISTRY, vol. 275, no. 4, 24 novembre 2000 (2000-11-24), pages 36703-707, XP002185747

## Description

La présente invention concerne l'utilisation de l'élément de réponse au récepteur ROR du gène IκBα, et tout spécialement de la séquence -910 / -898 du promoteur de ce gène, pour le criblage de substances utiles dans le traitement ou la prévention de l'inflammation. Dans une forme de mise en oeuvre particulière, l'invention concerne l'utilisation d'un récepteur ROR et/ou d'au moins un élément de réponse de celui-ci ou d'un équivalent fonctionnel de ceux-ci pour le criblage de substances utiles dans le traitement ou la prévention de l'inflammation.

Le complexe NF-κB est constitué d'une importante variété de dimères de protéines de la famille NF-κB/Rel. Cinq protéines de cette famille ont été identifiées chez les mammifères : NF-κB1 (p50 et son précurseur p105), NF-κB2 (p52 et son précurseur p100), c-Rel, RelA/p65 et RelB (Ghosh, S. et al., Annu. Rev. Immunol. 1998, 16: 225-60). Toute7s ces protéines comportent un domaine hautement conservé, appelé RHD pour (Rel homology domain), composé d'environ 300 acides aminés avec deux domaines « Ig-Like ». Ce domaine RHD est impliqué dans la dimérisation des protéines NF-κB, la fixation à l'ADN, l'interaction avec les protéines IκBs et possède un signal de localisation nucléaire (NLS). Les dimères NF-κB reconnaissent avec une affinité différentielle la séquence consensus GGGRNNYYCC, où R représente une base purique, Y représente une base pyrimidique et N représente une base quelconque (Miyamoto, S. Verma, I. M., Adv. Cancer Res. 1995, 66 : 255-92). Les protéines de la famille Rel/NF-κB présentent également une capacité différente à activer la transcription ; seules les protéines p65 et c-Rel en sont capables, bien que RelB active la transcription dans certains types cellulaires. L'invalidation des gènes codant pour les différentes protéines de cette famille a révélé des fonctions différentes spécifiques de chacun de ses membres. L'hétérodimère p50/p65 a été le premier dimère identifié et il est le plus abondant dans la plupart des types cellulaires.

La voie NF-κB régule la transcription d'un grand nombre de gènes impliqués dans les réponses immunes et inflammatoires (Bauerle, P.A. et Baichwal, V.R., Adv. Immunol. 1997, 65 : 111-37 ; Barnes, P.J. et Karin, M., N. Engl. J. Med. 1997, Ap 10, 336 (15) : 1066-71 ; Siebenlist, U. et al., Annu. Rev. Cell Biol. 1994, 10 : 405-55). Une dérégulation de cette voie de signalisation conduit à de multiples pathologies, comme des cancers, des maladies neurodégénératives, l'arthrite et l'asthme.

La voie de signalisation NF-κB est activée par un large spectre de stimuli : les cytokines (TNFα, IL-1), les mitogènes, le lipopolysaccharide, les virus, les protéines virales, l'ARN double brin, les stress physiques et chimiques (Ghost, S. et al., Annu. Rev. Immunol. 1998, 16: 225-60 ; Siebenlist, U. et al., Annu. Rev. Cell Biol. 1994, 10: 405-55).

Dans la plupart des cellules inactivées, NF-κB se trouve sous forme d'un complexe cytoplasmique inactif, par liaison avec une protéine inhibitrice IκB (Forman B.M. et Baltimore, D. Science 1998, 242: 540-546). L'activation de NF-κB se fait via l'activation d'une IκB kinase (IKK) qui phosphoryle la protéine IκBα sur la sérine 32 et la sérine 36, ce qui conduit à la dégradation d'IκBα et à la libération des protéines NF-κB p52 et p65 (Bauerle, P.A. et Baltimore D., Cell 1996, 87 : 13-20 ; Verma, I.M. Stevenson, J.K. Schwartz, E.M., Van Antwerp, D. et Miyamoto, S., Genes & development 1995, 9 : 2723-2735). Les dimères NF-κB ainsi libérés migrent jusqu'au noyau où ils régulent la transcription.

La famille IκB regroupe les protéines IκBα, IκBβ, IκBγ, IκBε, Bcl-3, NF-κB1, NF-κB2 (Whiteside, S.T. et Israel, A., Semin. Cancer Biol. 1997, Apr 8(2) : 75-82). Les protéines IkBs sont impliquées dans le processus terminal de la voie de signalisation NF-κB puisque ces protéines néosynthétisées sont dirigées dans le noyau et interagissent avec les complexes NF-κB fixés à l'ADN, entraînant le détachement du site de fixation et leur réexportation dans le cytoplasme (Arenzana-Seeisdedos, F. et al., J. Cell Sci. 1997, Feb. 110 : 369-78).

L'expression de la protéine IκBα est contrôlée par la voie NF-κB ce qui assure un rétrocontrôle de cette voie de signalisation (Sun, S.C. et al., Sciences 1993, Mar 26, 259 (5103) : 1912-5). Par ailleurs, les glucocorticoïdes semblent également contrôler l'expression IκBα (Auphan, N. et al. 1995, Oct. 13, 270 (5234) : 286-90 ; Scheinman, R.I., 1995, Oct. 13, 270(5234) : 283-6), alors que les oestrogènes réduisent son expression (Sun S.C. et al., Science 1993, Mar. 26, 259 (5103) : 1912-5). Enfin, l'analyse fonctionnelle du promoteur IκBα a montré le rôle crucial d'un site de liaison au facteur AP-1 (Kralova, J. et al., Oncogene 1996, Jun. 20, 12 (12) : 2595-604) et d'un site de liaison au facteur SP1 (Algarte, M. et al., Mol. Cell. Biol., 1999, Sep. 19 (9) : 64140-53).

Le récepteur RORα (Retinoid-related orphan receptor alpha) (NR1 F) est un membre de la famille des récepteurs nucléaires qui se lie sous forme de monomère, via un élément de réponse RORE composé d'une unique séquence de 6 paires de bases de motif hémipalyndromique AGGTCA précédé d'une région riche en A/T (Giguère, V., McBroom, L.B.D. & Flock, G. Molecular Cellular Biology, 1995, 15, 2517-2526 ; McBroom, L.B.D. & Flock, & Giguère, V. Molecular Cellular Biology, 1995, 15, 796-808). Dans certaines conditions RORα peut également se lier à l'ADN sous forme d'homodimère via un élément de réponse composé d'une répétition de deux motifs hémipalindromiques AGGTCA espacés de deux nucléotides et précédés d'une région riche en A/T (Harding, H.P. et al., Mol. Endocrinol. 1997, Oct 11 (11) : 1737-46).

Les sites RORE sont des éléments de réponse de ROR sur lesquels le récepteur ROR se fixe pour moduler l'activité transcriptionnelle du gène placé en aval. Ces sites peuvent être utilisés pour conférer une sensibilité pour ROR à un promoteur hétérologue.

Le gène RORα possède quatre isoformes ayant un domaine de liaison à l'ADN (DNA-binding domains DBD) commun et un domaine de liaison de ligands (Ligand-binding domains LBD) également commun. Ces isoformes diffèrent par leur domaine amino-terminal (Giguère, V. et al. Genes & Development 8, 538-553 (1994)). Les souris déficientes en RORα présentent des déficiences cérébrales (ataxie) caractéristiques des souris staggerer, qui portent une délétion au niveau du gène de RORα qui crée une protéine tronquée incapable de se lier à l'ADN (Hamilton, B.A. et al. Nature 1996, 379, 736-739t ; D'Ambrosio, D. et al. Journal of Clinical Investigation 1998, 101, 252-262 ; Dussault, I., Fawcett, D., Mathyssen, A., Bader, J.-A. et Giguère, V., Mechanisms of Development 1998, 70, 147-153 ; Steinmayr, M. et al. Journal of Biological Chemistry 1998, 95, 3960-3965). De plus, les souris staggerer souffrent d'une hyperproduction d'IL-1 (Kopmels, B., et al. Journal of Neurochemistry 1992 58, 192-199).

II a été montré dans l'art antérieur que la souris staggerer qui porte une mutation du gène RORα (mutation qui empêche la traduction du domaine de liaison à l'ADN) révèle des anomalies immunes comme l'hyperproduction d'IL-1α dans les macrophages (Kopmels, B., et al. Journal of Neurochemistry 1992 58, 192-199 ; Kopmels, B., et al. Journal of Neurochemistry 1990, 55 1980-1985).

Une autre étude a rapporté que le récepteur RORα, qui appartient à la famille des récepteurs nucléaires orphelins, est activé par le CGP 52608, dérivé apparenté aux thiazolidinediones, dans les cellules SL3 de drosophile (Wiesenberg, I. et al. Molecular Pharmacology 1998, 53, 1131-1138). Cette étude a aussi montré que le CGP 52608 possède des activités anti-arthritiques *in vivo*. Elle n'apporte cependant aucun élément prouvant que l'activité anti-inflammatoire de CGP 52608 dépend de ROR dans les cellules de mammifère.

II a par ailleurs été suggéré, seulement sur la base de la présence d'un élément de réponse à ROR dans son promoteur, que le gène codant pour la 5-lipoxygénase, enzyme importante impliquée dans le contrôle des réactions inflammatoires et allergiques, constituait une cible de RORα (Steinhilber et al. 1995, JBC 270 (13) : 7037-7040).

Les travaux de recherche réalisés dans le cadre de la présente invention ont montré une nouvelle fonction du récepteur RORα1. De manière surprenante, les inventeurs ont prouvé pour la première fois que RORα1 régule de façon négative la réponse inflammatoire en interférant avec la voie NF-κB. En effet, il est maintenant démontré que la transcription d' IκBα, principale protéine inhibitrice de la voie NF-κB, est activée par RORα1 via un nouvel élément de réponse RORE, identifié dans le promoteur du gène IκBα. Cet élément de réponse correspond à un site RORE consensus parfait et se situe entre les positions -910/-898 du promoteur IκBα, la séquence du site étant gagcacAATGTAGGTCAgatag (Ito, C.Y., Kazantsev, A.G. et Baldwin, A.S., Nucleic Acid Research, 1994 22, 3787-3792).

Par ailleurs, les inventeurs ont démontré que la mutation de cette séquence se traduit par une perte de l'effet de RORα1, ce qui illustre son importance fonctionnelle. Tout autre récepteur nucléaire ou toute autre substance qui se lierait à cette séquence est donc susceptible de moduler l'expression d' IκBα.

Or, la protéine IκBα en se liant au facteur nucléaire NF-κB forme un complexe inactif (IκBα-NF-κB) qui empêche l'activation de l'expression de gènes proinflammatoires comme les gènes de certaines cytokines ou le gène COX-2 ; Ces données prouvent que RORα1 ou tout autre facteur nucléaire qui se lie à la séquence -910/-898 du promoteur IκBα est une cible potentielle pour le traitement des maladies inflammatoires chroniques.

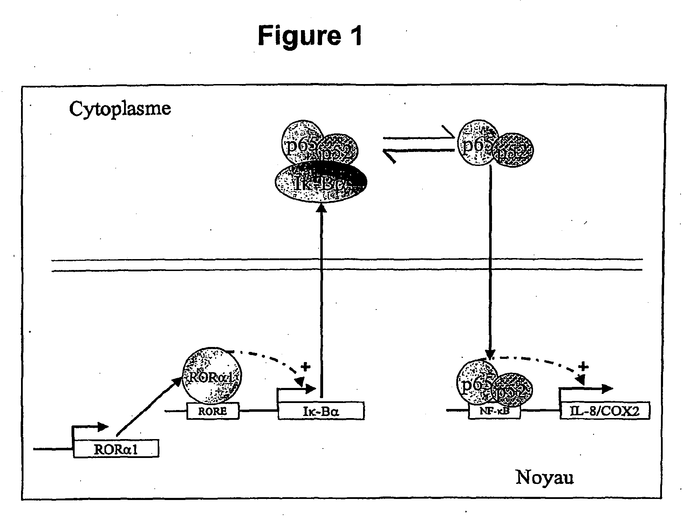

La figure 1 en annexe est une représentation schématique des mécanismes mis en jeu dans la voie de régulation de la réponse inflammatoire identifiés dans le cadre de la présente invention.

La mise en évidence du rôle de la séquence -910/-898 a donc permis de mettre au point de nouvelles méthodes d'identification de substances utiles pour le traitement des maladies inflammatoires (chroniques) humaines comme l'athérosclérose et l'arthrite rhumatoïde. II est entendu que la séquence identifiée par les inventeurs peut également être utilisée pour identifier des nouveaux facteur nucléaires qui affectent l'activité du promoteur IκB via cette séquence et par conséquent, peuvent être utiles pour le traitement des maladies inflammatoires (chroniques) humaines comme l'athérosclérose et l'arthrite rhumatoïde.

La mise en évidence d' IκBα en tant que cible de RORα1 a donc également permis de mettre au point de nouvelles méthodes d'identification de substances utiles pour le traitement des maladies inflammatoires (chroniques) humaines, comme l'athérosclérose et l'arthrite rhumatoïde.

La présente invention a donc pour objet un procédé d'identification de substances utiles pour le traitement de l'inflammation, caractérisé par le fait qu'il comprend les étapes suivantes :
a) la mise en contact de ladite substance avec une construction d'acide nucléique comprenant l'élément de réponse au récepteur ROR du promoteur du gène IκBα, dans des conditions susceptibles de permettre à ladite substance de se fixer sur ledit élément de réponse,
b) la mesure de la fixation éventuelle de ladite substance sur l'élément de réponse ou de l'activité transcriptionnelle de l'élément de réponse ou d'un promoteur le contenant,
c) éventuellement la comparaison de la mesure de l'étape (b) avec une mesure réalisée dans les mêmes conditions qu'aux étapes (a) et (b) mais avec une construction d'acide nucléique comprenant l'élément de réponse muté.

De préférence l'élément de réponse est l'élément de réponse au récepteur RORα et de préférence au récepteur hRORα.

Selon une forme particulière de réalisation du procédé de l'invention, les conditions susceptibles de permettre à ladite substance de se fixer sur ledit élément de réponse comprennent la présence du récepteur ROR ou un équivalent fonctionnel de celui-ci.

Ainsi, le procédé de l'invention comprend les étapes suivantes :
a) la mise en contact de ladite substance avec le récepteur ROR et une construction d'acide nucléique comprenant l'élément de réponse audit récepteur du promoteur du gène IκBα, dans des conditions susceptibles de permettre audit récepteur , à ladite substance ou au complexe formé du récepteur , et de ladite substance de se fixer sur ledit élément de réponse,
b) la mesure de la fixation éventuelle du récepteur ROR, de la substance ou d'un complexe formé du récepteur ROR et de ladite substance sur l'élément de réponse ou de l'activité transcriptionnelle de l'élément de réponse ou d'un promoteur le contenant,
c) éventuellement la comparaison de la mesure de l'étape (b) avec une mesure réalisée dans les mêmes conditions qu'aux étapes (a) et (b) mais avec une construction d'acide nucléique comprenant l'élément de réponse muté.

Ainsi, au sens de la présente invention, on désigne par récepteur ROR, l'ensemble des isoformes α, β et γ de la famille ROR. On préfère plus particulièrement le récepteur RORα et tout préférentiellement le récepteur hRORα.

Une forme avantageuse de réalisation du procédé de l'invention, consiste à mettre en oeuvre à l'étape (a) une construction d'acide nucléique qui comprend plusieurs copies de l'élément de réponse au récepteur RORα et de préférence hRORα du promoteur du gène IκBα. La construction peut par exemple comprendre de 1 à 10 copies de l'élément de réponse au récepteur RORα, typiquement de 1 à 5 copies.

Une forme avantageuse de mise en oeuvre de l'invention consiste à utiliser à l'étape (a) une construction d'acide nucléique comprenant tout ou partie du promoteur du gène IκBα contenant l'élément de réponse au récepteur ROR.

Un exemple préféré de construction d'acide nucléique selon l'étape (a) comprend ou est constituée par la séquence entre les positions -910/-898 du promoteur IκBα répondant à la formule suivante :
GAGCACAATGTAGGTCAGATAG (SEQ ID No : 1)
ou un fragment de celle-ci contenant mais pas limité à la séquence consensus de 6 paires de bases soulignée ci-dessus. Des exemples de constructions d'éléments de réponse au récepteur ROR utilisables dans l'invention comprennent les séquences particulières suivantes :
TAGGTCAG (SEQ ID NO: 6)
GTAGGTCAGA (SEQ ID NO: 7)
ATGTAGGTCAGATA (SEQ ID NO: 8)

Dans un mode particulier, on utilise un acide nucléique comprenant l'ensemble de la séquence SEQ ID NO : 1. Cette séquence présente l'avantage, par rapport à la seule séquence consensus de 6 paires de bases, soulignée dans SEQ ID No : 1 ci-dessus, de couvrir une ou plusieurs régions impliquées dans les interactions avec les facteurs nucléaires.

La mesure de la fixation éventuelle de la substance, du récepteur ROR ou d'un complexe formé du récepteur ROR et de ladite substance sur l'élément de réponse peut être effectuée par toute méthode connue de l'homme du métier par exemple en détectant un signal produit par l'élément de réponse suite à ladite fixation. II peut s'agir de toutes méthodes directes ou indirectes, comme celles utilisant un gène rapporteur, des tests de liaison, etc..

Ainsi, une forme préférée de mise en oeuvre de l'invention consiste à utiliser une construction d'acide nucléique combinant l'élément de réponse du gène IκBα, avec un gène rapporteur. Avantageusement, ledit gène rapporteur est placé sous le contrôle d'un promoteur comprenant au moins une copie dudit élément de réponse.

Le gène rapporteur peut donc être placé sous le contrôle de tout promoteur dont la séquence comprend la séquence SEQ. ID. NO : 1 ou un variant fonctionnel de celle-ci. Cette séquence particulière peut être présente à raison d'une ou de plusieurs copies dans le promoteur (préférentiellement 1 à 10 et encore plus préférentiellement 1 à 6), en amont ou en aval ou en interne, dans la même orientation ou dans l'orientation opposée. Dans un mode préféré de mise en oeuvre de l'invention, le gène rapporteur est placé sous le contrôle d'un promoteur qui comprend une ou plusieurs copies de la séquence SEQ ID NO : 1 ou d'un fragment de celle-ci comprenant au moins la région consensus. Préférentiellement, il s'agit d'un promoteur dont le différentiel d'activité en l'absence et en présence de ROR ou d'un équivalent fonctionnel peut être détecté.

Pour la réalisation d'un promoteur de l'invention, l'élément de réponse au récepteur ROR peut être associé à un promoteur minimal transcriptionnel. Le promoteur minimal est un promoteur transcriptionnel ayant une activité basale faible ou inexistante, et susceptible d'être augmentée en présence d'un activateur transcriptionnel (e.g., ROR). Un promoteur minimal peut donc être un promoteur naturellement faible dans les cellules de mammifère, c'est-à-dire produisant une expression non toxique et/ou non suffisante pour obtenir un effet biologique prononcé. Avantageusement, un promoteur minimal est une construction préparée à partir d'un promoteur natif, par délétion de région(s) non essentielle(s) à l'activité transcriptionnelle. Ainsi, il s'agit de préférence d'un promoteur comprenant essentiellement une boîte TATA, généralement d'une taille inférieure à 160 nucléotides, centrée autour du codon d'initiation de la transcription. Un promoteur minimal peut ainsi être préparé à partir de promoteurs viraux, cellulaires, forts ou faibles, tels que par exemple le promoteur du gène de la thymidine kinase (TK) du virus de l'herpès, le promoteur immédiat du CMV, le promoteur PGK, le promoteur SV40, etc..

Dans un mode de réalisation préféré, le gène rapporteur est placé sous le contrôle du promoteur du gène IκBα.

Tout gène rapporteur permettant de mesurer l'activité de récepteurs nucléaires sur la séquence comprenant leur élément de réponse peut être utilisé dans le procédé de criblage selon l'invention. Parmi ceux-ci, on peut citer par exemple le gène de la chloramphénicol acétyltransférase (CAT), le gène de la luciférase de luciole (Luc) ou de Renilla (Ren), le gène de la phosphatase alcaline secrétée (PAS) ou celui de la bêta-galactosidase (β-Gal) et le gène IκBα lui-même.

L'activité des protéines codées par ces gènes peut être facilement mesurée par des méthodes classiques et permet de connaître indirectement l'effet des récepteurs nucléaires sur l'expression des gènes en mesurant la quantité de protéines produites et/ou leur activité enzymatique.

Selon une forme de réalisation toute particulière de l'invention, le gène rapporteur est le gène IκBα. Dans ce cas, l'accumulation de la protéine IκBα peut être mesurée par sa capacité à lier NF-κB ou à empêcher son action par exemple en utilisant un vecteur rapporteur qui comporte plusieurs éléments de réponse à NF-κB cloné en amont d'un gène rapporteur.

La mise en oeuvre d'un gène rapporteur présente l'avantage de vérifier si le récepteur ou un équivalent fonctionnel de celui-ci ou ladite substance est capable non seulement de se fixer, seule ou complexée avec le récepteur ROR, sur ledit élément de réponse, mais aussi d'augmenter l'activité transcriptionnelle de l'élément de réponse ou d'un promoteur comprenant ledit élément de réponse. Il est ainsi possible de confirmer le rôle de ladite substance dans l'inhibition de la réponse inflammatoire.

Avantageusement cette capacité d'augmentation de l'activité transcriptionnelle peut être évaluée à l'étape (b) et éventuellement (c) sur le gène IκB par toutes méthodes directes ou indirectes connues de l'homme du métier, comme une transfection, une analyse des ARNm ou de la protéine *in vitro* et sur les modèles *in vitro* et *in vivo*.

Un exemple de procédé selon l'invention comprend les étapes suivantes :
a) la mise en contact de ladite substance avec un hôte cellulaire transformé par une construction d'acide nucléique comprenant au moins un gène rapporteur combiné de manière fonctionnelle à l'élément de réponse au récepteur ROR du promoteur du gène IκBα comme défini précédemment,
b) la mesure de l'expression du gène rapporteur par tout moyen approprié,
c) éventuellement la comparaison de la mesure de l'étape (b) avec une mesure réalisée dans les mêmes conditions qu'aux étapes (a) et (b) mais avec une construction d'acide nucléique comprenant l'élément de réponse muté.

Selon une forme de réalisation particulière du procédé ci-dessus, l'hôte cellulaire de l'étape (a) exprime un récepteur ROR ou un équivalent fonctionnel de celui-ci comme défini précédemment.

A l'étape (c), la comparaison de la mesure de l'étape (b) est avantageusement effectuée avec une mesure réalisée dans les mêmes conditions qu'aux étapes (a) et (b) mais avec une construction d'acide nucléique comprenant l'élément de réponse muté, c'est-à-dire un élément de réponse au ROR dont la séquence a été modifiée pour le rendre incapable de lier le récepteur ROR de manière fonctionnelle. Un tel élément de réponse muté répond avantageusement à la séquence suivante : GAGCACAATGTXXXXXXGATAG, dans laquelle X est choisi parmi A, T, C, G. A titre d'exemple non limitatif, on préfère les séquences mutées suivantes :
GAGCACAATGTATTTCAGATAG (SEQ ID NO : 9)
GAGCACAATGTAAATCAGATAG (SEQ ID NO :10)
GAGCACAATGTACATCAGATAG (SEQ ID NO : 11)
GAGCACAATGTAACTCAGATAG (SEQ ID NO : 12)
GAGCACAATGTATATCAGATAG (SEQ ID NO : 13)
GAGCACAATGTAATTCAGATAG (SEQ ID NO : 14)
GAGCACAATGTACCTCAGATAG (SEQ ID NO : 15)

Les substances susceptibles d'être identifiées par le procédé selon l'invention peuvent être des composés chimiques ou biologiques. II peut ainsi s'agir de facteurs nucléaires.

En outre, les méthodes classiques pour identifier des clones codant pour des protéines qui lient l'ADN peuvent être utilisées dans le procédé de l'invention. Par exemple, il est possible d'utiliser le criblage d'une banque d'expression dans λgt11 (Ausubel et al. Current Protocols in Molecular Biology, John Wiley & Sons, Inc. 1997), la purification par chromatographie d'affinité suivie du séquençage de la protéine (Ausubel et al. Current Protocols in Molecular Biology, John Wiley & Sons, Inc. 1997), la méthode dite de « one hybrid » (Wang, M.M., Nature 1993, Jul. 8, 364 (6433) : 121-6), ou encore la méthode dite de « phage display » (Smith, G.P. et Scott, J.K., Methods Enzymol. 1993, 217† : 228-57 ; O'Neil, K.T. et Hoess, R.T., Curr. Opin Struct. Biol., 1995, Aug. 5(4) † : 443-9).

Un vecteur comprenant au moins une construction d'acide nucléique comprenant au moins une copie de l'élément de réponse du gène IκBα muté ou non, susceptible d'être utilisé dans un procédé d'identification de substance décrit précédemment est aussi dévoilé

Des hötes celluaires peuvent comprendre un tel vecteur. On entend par hôte cellulaire, tout type cellulaire adapté à l'expression des gènes ci-dessus, comme notamment des cellules de mammifères, des bactéries ou des levures ou encore des cellules d'insectes. Les cellules hôtes peuvent être des cellules de mammifères (hépatocytes, fibroblastes, cellules endothéliales, musculaires, etc.). Ces cellules peuvent être des cellules, humaines. II peut également s'agir de cultures primaires ou de lignées établies. Dans un autre mode de mise en oeuvre, il est possible également d'utiliser des cellules procaryotes (bactéries), des cellules de levure (Saccharomyces, Kluyveromyces, etc.), des cellules végétales, etc.

Les composés peuvent être mis au contact des cellules à différents moments, selon leur(s) effet(s), leur concentration, la nature des cellules et l'appréciation technique.

Les vecteurs utilisés sont, bien entendu, adaptés au type cellulaire transfecté, on peut citer des plasmides, des virus ou des chromosomes artificiels.

La substance testée peut être mise en contact avec la construction d'acide nucléique ou les cellules sur (ou dans) tout support approprié et notamment sur une plaque, dans un tube ou une flasque, une membrane, etc.. Généralement, la mise en contact est réalisée dans une plaque multipuits ce qui permet de conduire, en parallèle, des essais nombreux et variés. Parmi les supports typiques on trouve des plaques de microtitration et plus particulièrement des plaques 96 ou 384 puits (ou plus), faciles à manipuler.

Selon le support et la nature du composé test, des quantités variables de cellules peuvent être utilisées lors de la mise en oeuvre des méthodes décrites. De manière classique , 10³ à 10⁶ cellules sont mises en contact avec un type de composé test, dans un milieu de culture approprié, et de manière préférentielle entre 10⁴ et 10⁵ cellules.

La quantité (ou la concentration) de composé test peut être ajustée par l'utilisateur selon le type de composé (sa toxicité, sa capacité de pénétration cellulaire, etc.), le nombre de cellules, la longueur de la période d'incubation, etc. Généralement, les cellules sont exposées à des quantités de composés test qui varient de 1 nM à 1 mM. II est bien sûr possible de tester d'autres concentrations sans dévier de la présente invention. Chaque composé peut, de plus, être testé, en parallèle, à différentes concentrations. Par ailleurs, différents adjuvants et/ou vecteurs et/ou produits facilitant la pénétration des composés dans les cellules tels que des liposomes, des lipides cationiques ou des polymères peuvent, en outre, être utilisés, si nécessaire. Le contact peut être maintenu par exemple entre quelques minutes et plusieurs heures ou jours, particulièrement entre 5 et 72 heures, généralement entre 12 et 48 heures. Les cellules et les divers réactifs doivent, de préférence, rester en contact suffisamment longtemps pour permettre la synthèse de novo du produit d'expression du gène rapporteur ou l'interaction étudiée.

L'invention est illustrée par les exemples qui suivent et qui se réfèrent aux dessins en annexe dans lesquels :
Les figures 2A et 2B illustrent l'expression de RORα dans différents types cellulaires de la paroi vasculaire selon une analyse par RT-PCR (35 cycles) de l'ARNm de RORα (Figure 2A) et de GAPDH (Figure 2B) (C-PCR et C-RT sont les contrôles négatifs pour la PCR et la RT respectivement).
La figure 2C montre l'analyse par RT-PCR de l'ARNm de RORα dans les cellules musculaires lisses infectées par Ad-RORα1 ou Ad-GFP pendant 24 heures.
La figure 2D montre l'analyse de l'expression de la protéine RORα où les cellules musculaires lisses infectées pendant 24 heures avec ou sans Ad-RORα1.
   Les expériences d'immunocytochimie sont conduites comme décrit précédemment (Chinetti, G. et al. Journal of Biological Chemistry, 1980, 273, 25573-25580) par utilisation d'un anticorps RORα polyclonal de lapin contre les acides aminés 163 à 225.
Les figures 3A et 3B montrent que RORα1 inhibe la sécrétion de IL-6 (figure 3A) et IL-8 (figure 3B) et l'expression de COX-2 induites par TNFα dans les cellules musculaires lisses. Les cellules musculaires lisses primaires sont infectées avec les virus Ad-RORα1 et Ad-GFP pendant 16 heures, puis stimulées par 24 heures par du TNFα (10 ng/ml). A la fin du traitement les concentrations en IL-6 et IL-8 dans le milieu sont déterminées par ELISA (R&D systems UK).
Les figures 3C et 3D illustrent l'expression des protéines COX2 et RORα mesurée par analyse par Western blot. Les niveaux de protéines COX2 sont normalisés vis-à-vis du contenu cellulaire en protéine -actine (NS = non spécifique).
Les figures 4A à 4C illustrent l'inhibition de l'activation de NF-κB par RORα1.

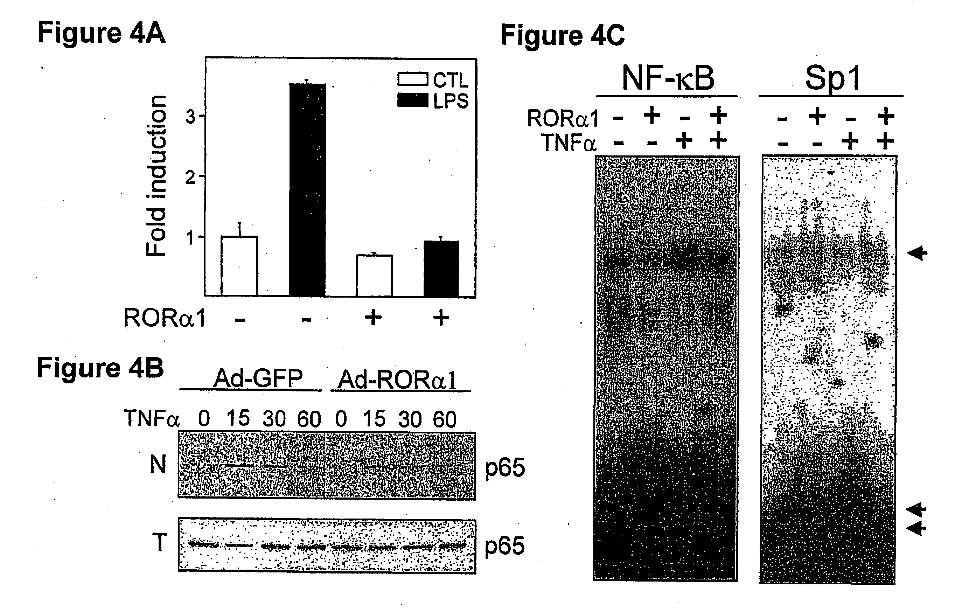

La figure 4A montre des cellules PAC1A transfectées par le gène reporteur (NF-κB) 3-Luc (100 ng) en présence d'un vecteur qui permet la sur-expression de RORα1 (PSG5-RORα1; 50 ng) ou d'un vecteur vide (PSG5 contrôle). Deux heures après la transfection, les cellules sont nourries avec du milieu DMEM complété par 0,2 % de FCS en présence ou non de LPS (10 mg/ml) pendant 24 heures.
La figure 4B montre des cellules musculaires lisses primaires infectées avec Ad-RORα1 et Ad-GFP pendant 16 heures, puis stimulées pendant 0, 15, 30 et 60 minutes avec TNFα (10 ng/ml). Les extraits protéiques totaux (T; gel inférieur) et nucléaire (N ; gel supérieur) sont préparés et analysés par Western blot utilisant un anticorps anti-p65 (Santz Cruz).
La figure 4C montre des cellules musculaires lisses primaires infectées avec Ad-RORα1 et Ad-GFP pendant 16 heures, puis stimulées pendant 30 minutes avec TNFα (10 ng /ml). Les extraits protéiques nucléaires (5 mg) sont préparés et analysés par EMSA utilisant des sondes marquées NF-κB et Sp1 (Promega). Les complexes ayant migré sont indiqués par une flèche. La double flèche indique la sonde libre.

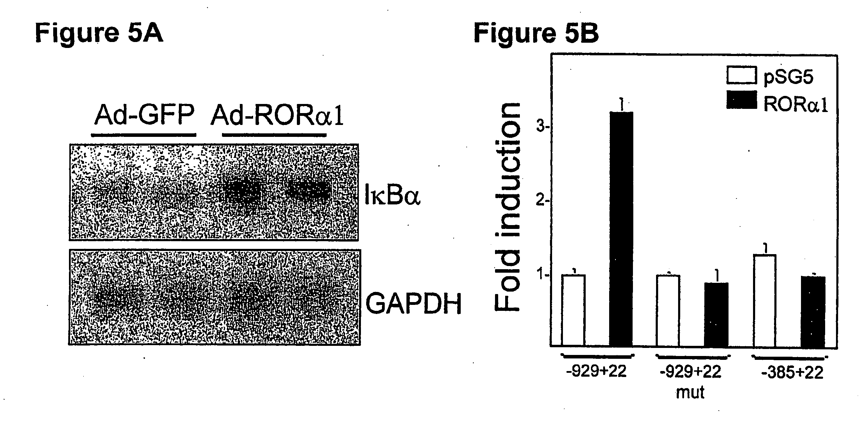

Les figures 5A et 5B illustrent l'activation de la transcription d'IκBα induite par le récepteur RORα1 dans un vecteur qui permet la sur expression de RORα (PSG5-RORα1).
La figure 5A montre les cellules musculaires lisses primaires infectées avec Ad-RORα1 et Ad-GFP pendant 16 heures. L'ARN total est préparé, les niveaux en ARNm d'IκBα et GAPDH sont mesurés par Northem blot.
La figure 5B montre les cellules PAC1A transfectées avec des fragments du promoteur d'IκBα (100 ng) en présence de PSG5-RORα1 ou du vecteur vide (PSG5 50 ng).

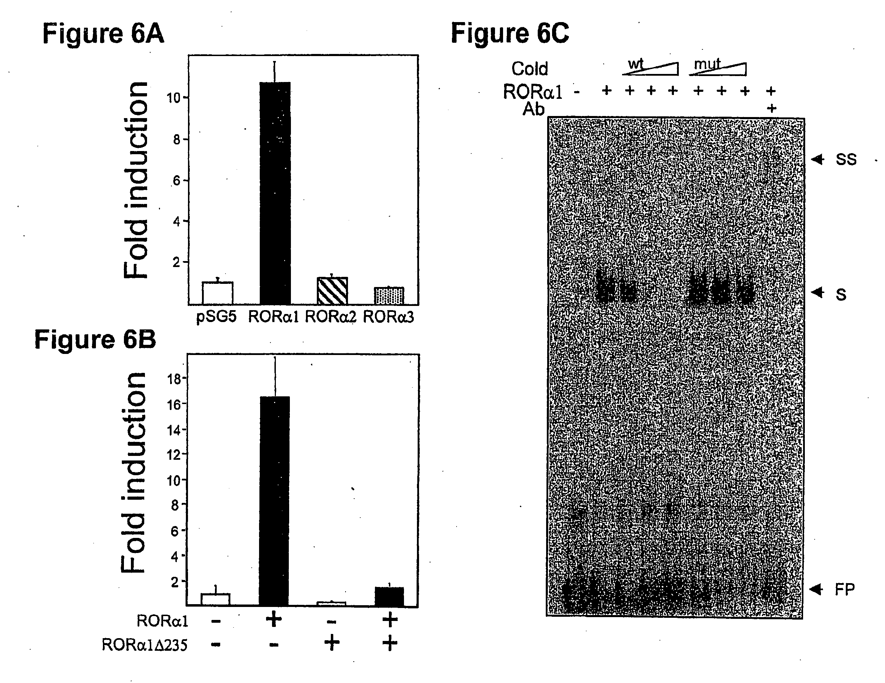

Les figures 6A à 6C illustrent l'activation spécifique de la transcription d'IκBα par RORα1.
   RORα1 se lie à un site RORE à l'intérieur du promoteur d'IκBα mais pas RORα2 ni RORα3 ;
La figure 6A montre les cellules PAC1A transfectées avec le vecteur reporteur (IκBα-RORE) 2-TK-Luc (100 ng) en présence de RORα1, de RORα2 ou de RORα3 (50 ng) ou du vecteur vide PSG5 (50 ng).
La figure 6B montre les cellules PAC1A transfectées avec le vecteur (IκBα-RORE) 2-TK-Luc (100 ng) en présence de RORα1 (50 ng) et de RORα1Δ235 ou du vecteur vide (50 ng).
La figure 6C illustre des expériences EMSA conduites par utilisation de la protéine traduite in vitro RORα1 (TNT-T7 Promega) et une sonde marquée IκBα-RORE. Des concentrations croissantes (1x, 10x et 100x) du compétiteur oligonucléotidique natif (AGGTCA) ou muté (ACCTCA) sont utilisées pour démontrer la spécificité du complexe migrant. Afin d'augmenter la mobilité du complexe, un µl de l'anticorps RORα est ajouté à la réaction de liaison (S : migration du complexe ; SS : super-migration du complexe ; FP : sonde libre).

### I - Matériel et méthodes.

### 1) Culture cellulaire.

Des cellules musculaires lisses d'aorte humaine (PromoCell, Heidelberg, Allemagne) et des cellules endothéliales d'artère coronaire humaine (Clonetics) ont été maintenues en culture primaire dans des conditions habituelles. Les cellules ont été utilisées entre les passages 5 à 8. Des monocytes et des macrophages humains ont été préparés et maintenus en culture primaire comme décrit précédemment.

### 2) Analyses de ARNs.

La préparation et l'analyse par Northern Blot des ARNs ont été réalisées comme décrit précédemment à l'aide des fragments des cDNA IκBα et GAPDH utilisés comme sondes. Pour l'analyse de l'expression de RORα par RT-PCR, l'ARN total a été transcrit par la transcriptase inverse et ensuite amplifié par PCR à l'aide des amorces suivantes :
- pour RORα, 5'-GTCAGCAGCTTCTACCTGGAC-3' (SEQ ID No : 2) et 5'-GTGTTGTTCTGAGAGTGAAAGGCACG-3' (SEQ ID No : 3) (taille du fragment : 482 pb) ;
- pour GAPDH, 5'-ATGCAGCCCCGAATGCTCCTCATCGTGGCC-3' (SEQ ID No : 4) et 5'-TTCTTGGAGGCCATGTGGGCCAT-3' (SEQ ID No : 5) (taille du fragment : 239 pb)

### 3) Production des adénovirus.

Les adénovirus recombinants Ad-GFP et Ad-RORα1 ont été obtenus par recombinaison homologue dans E. Coli après insertion des cDNAs dans le vecteur pADCMV2. Les stocks de particules virales ont été obtenus comme décrit précédemment. Le titre de la suspension a été déterminé par comptage des plages de lyse après infection de cellules 293 et défini comme pfu/ml. Les cellules ont été infectées à un multiplicité d'infection de 100 particules virales par cellules en ajoutant les stocks viraux directement dans le milieu de culture des cellules musculaires lisses.

### 4) Plasmides et transfection transitoire.

Les vecteurs d'expression pSG5-hRORα1, -hRORα2, -hRORα3 et -RORαΔ235 ont été dérivés des vecteurs pCMX précédemment décrits. Un fragment correspondant à la région -929 /+22 du promoteur IκBα a été amplifié à partir d'ADN génomique humain et inséré dans le plasmide pGL2 basique de Promega pour donner la construction p(-922/+22) IκBα Luc. La mutation du site ROR présent dans ce fragment (AGGTCA muté en ACCTCA) a été introduite par mutagenèse dirigée (Stratagène). Le vecteur rapporteur p(-385/+22) IκBα a été offert par le Dr. Israël (Institut Pasteur de Paris). Le vecteur rapporteur (IκBα-RORE) 2-Tk-Luc a été créé par l'insertion de deux copies du site RORE du promoteur IκBα en amont du promoteur minimal de la thymidine kinase du virus de l'Herpès Simplex. La construction (NF-κB) 3-Luc a été obtenue auprès de Stratagène. Les cellules PAC1A (une lignée de cellules musculaires lisse d'aorte de rat) ont été transfectées à l'aide d'un lipide cationique comme décrit précédemment.

### II - Résultats.

Compte tenu du fait que l'inflammation chronique est une caractéristique de l'athérosclérose (Ross, R. New England Journal of Medicine 340, 115-126 (1999)) et que les souris staggerer présentent un profil pro-inflammatoire, les inventeurs ont déterminé l'expression de ROR dans différents types cellulaires de la paroi vasculaire par RT-PCR.

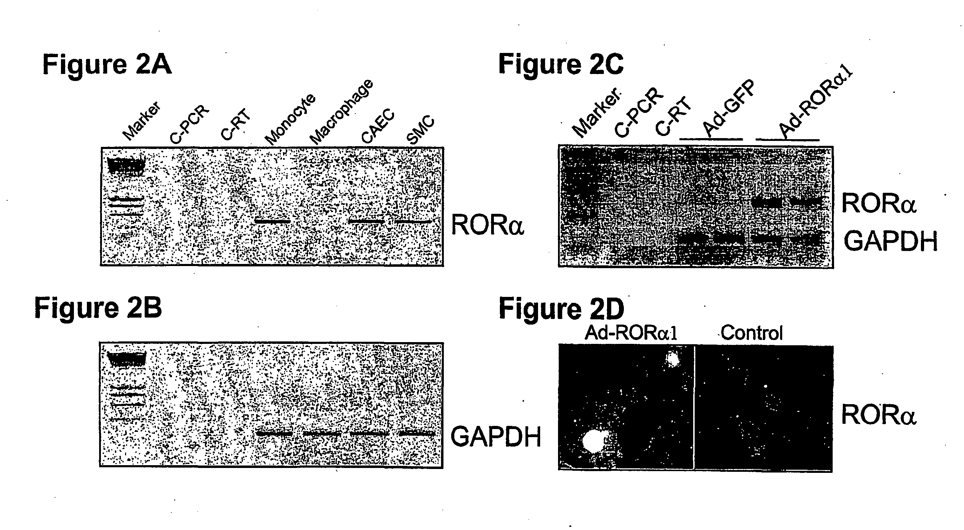

L'expression de RORα a été détectée dans les cellules endothéliales, les cellules musculaires lisses (CML) et les monocytes (figure 2A). Par contre, il a été mis en évidence que l'expression de RORα est beaucoup plus basse dans les macrophages issus de monocytes.

Afin de regarder le rôle potentiel de RORα dans le contrôle de la réponse inflammatoire, les inventeurs ont généré par recombinaison homologue un adénovirus codant pour l'isoforme RORα1, qui est prépondérante (Forman, B.M et al. Molecular Endocrinology 8, 1253-1261 (1994)). L'infection des CML primaires par Ad-RORα1 induit l'expression de RORα, alors que l'infection par Ad-GFP utilisé comme contrôle négatif n'affecte pas les niveaux endogènes (figure 2C). Les expériences d'immunocytochimie utilisant les anticorps polyclonaux dirigés contre RORα (aa 163-225) ont confirmé ces résultats au niveau de la protéine et ont de plus démontré qu'à la fois RORα endogène et RORα exogène sont principalement localisés dans le noyau (figure 2D).

Ensuite, l'influence de RORα1 sur les différents marqueurs de la réponse inflammatoire vasculaire induite par TNFα a été évaluée.

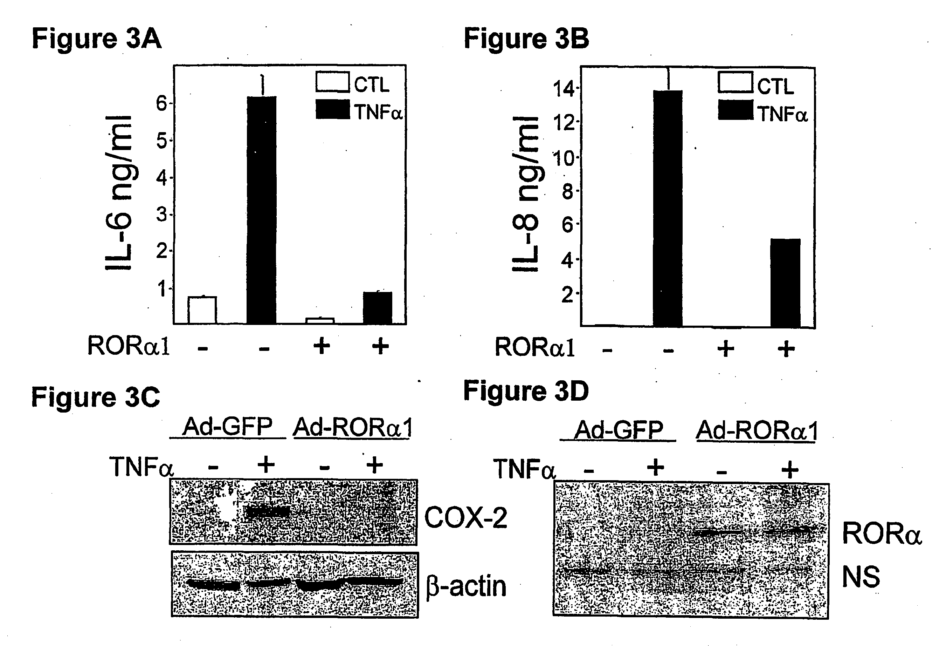

En absence de stimulation, à la fois IL-6 et IL-8 ont été détectés dans les cellules infectées par Ad-GFP, ce qui est dû à l'immunogénicité de la construction. Cette faible induction est, de façon significative, réprimée par l'infection par Ad-RORα1 (figures 3A et 3B). Le traitement par TNFα (24 h) des cellules infectées par Ad-GFP conduit à une induction marquée des deux cytokines qui peut être réprimée de façon significative par l'infection par Ad-RORα1 (figures 3A et 3B). Dans les cellules Ad-GFP nous n'avons pas détecté de protéines endogènes RORα ce qui est certainement dû à la faible affinité de l'anticorps utilisé. Cependant, dans les cellules infectées par Ad-RORα1, une expression accrue de RORα a été détectée, dont le niveau n'a pas été affecté par la stimulation par le TNFα (figure 3C). Dans les mêmes expériences, les niveaux en protéines COX-2 ont été contrôlés. Le TNFα induit fortement l'expression de COX-2, effet qui est complètement diminué dans les cellules infectées par Ad-RORα1 (figure 3D). Ces données prouvent que RORα1 régule de façon négative la réponse inflammatoire induite par le TNFα dans les cellules musculaires lisses primaires aortiques.

Dans le cadre des recherches ayant conduit à la mise en oeuvre de l'invention, les inventeurs ont transfecté transitoirement des cellules PAC1A (lignée de cellules musculaires lisses de rats) par un promoteur sous le contrôle de la voie NF-κB, en présence ou non de RORα1. Compte tenu du fait que ces cellules ne répondent pas à la stimulation par TNFα, les inventeurs ont utilisé le LPS comme inducteur de la voie NF-κB. Le traitement par le LPS augmente de façon significative (plus de 3 fois) l'activité du promoteur sous le contrôle de NF-κB. Cette induction est réduite lors de la co-transfection par RORα1. L'activité promotrice de base n'est pas affectée de façon significative dans ces conditions (figure 4A). Ces résultats confirment que RORα1 exerce son activité anti-inflammatoire par inhibition de l'activité transcriptionnelle de NF-κB.

Les inventeurs ont ensuite testé l'influence de la surexpression de RORα1 sur la translocation nucléaire de p65 par Western blot. Ils ont constaté que le traitement par TNFα conduit à une translocation nucléaire transitoire de p65 dans les cellules Ad-GFP. Cette translocation est moins prononcée dans les cellules infectées par Ad-RORα1, ce qui indique que RORα1 contrôle la translocation de p65 sans réguler l'expression totale de la protéine p65 (figure 4B). Des expériences de retard sur gel (EMSA) ont été conduites afin de regarder les conséquences de cette réduction de migration. Vingt-quatre heures après l'infection des cellules, du TNF est ajouté pendant 30 minutes. A la fin de cette période de traitement, des extraits nucléaires sont préparés et utilisés pour évaluer la présence des protéines NF-κB p50 et p65 par des expériences de retard sur gel qui utilisent des sondes marquées dont les séquences correspondent au site consensus de NF-κB. Les cellules Ad-GFP révèlent une activité de liaison sur le site NF-κB qui est significativement plus basse que dans les cellules infectées par Ad-RORα1, ce qui va dans le sens des résultats précédents (figure 3A et 3B). Les inventeurs ont constaté que la stimulation par du TNFα conduit à une forte augmentation de la liaison de NF-κB à son élément de réponse, liaison qui est fortement réduite dans les cellules Ad-RORα1. Ni le TNFα, ni la surexpression de RORα1 n'affectent la liaison de facteurs nucléaires sur une sonde qui correspond au site Sp1 (figure 4C), ce qui indique que l'action de RORα1 est spécifique de la voie NF-κB.

Par ailleurs, il est connu que nombre de récepteurs nucléaires inhibent les transcriptions contrôlées par NF-κB et la liaison au site NF-κB, par interaction avec la sous-unité p65 (Gôttlicher, M. Heck, S. & Herrlich, P. Journal of Molecular Medicine 76, 480-489 (1998)). Il a ainsi été montré que la cotransfection par p65 conduit à une forte induction du promoteur contrôlé par NF-κB dans les cellules PAC1A (données non fournies). Cette induction n'est pas affectée par la cotransfection par RORα1, ce qui indique que, contrairement à d'autres récepteurs nucléaires, RORα1 interfère de façon négative avec la voie NF-κB sans s'associer à p65.

Dans les cellules au repos, la protéine IκBα séquestre l'hétérodimère p50/p65 sous forme d'un complexe cytoplasmique inactif. Etant donné que RORα inhibe la translocation de p65 et sa liaison consécutive au site NF-κB, les niveaux d'expression de IκBα ont été analysés à la fois dans les cellules infectées par Ad-GFP ou Ad-RORα1. Les cellules Ad-GFP expriment des niveaux bas d'ARNm d'IκBα (figure 5A). Les niveaux en transcrit IκBα sont augmentés de façon importante dans les cellules infectées par Ad-RORα1 (figure 5A). Ceci indique que RORα1 inhibe la voie NF-κB par induction de l'expression du gène IκBα.

Le fragment du promoteur du gène IκBα compris entre les positions -929 et +22 a été amplifié par PCR et inséré en amont du gène rapporteur luciférase (Ito, C.Y., Kazantsev, A.G. & Baldwin, A.S. Nucleics Acid Research 22, 3787-3792 (1994)). Cette construction est à la base de la présente invention. La cotransfection de ce vecteur avec un vecteur d'expression de RORα1 induit une augmentation de l'activité luciférase dirigée par le promoteur -929/+22 mais pas par le promoteur -385/+22. Ceci indique que la région de régulation par RORα1 se situe entre les positions -929 et -385 (figure 5B). Une analyse de séquence de cette région montre la présence d'un élément de réponse (RORE) dans cette région. La mutation de cet élément de réponse RORE bloque l'effet de RORα (figure 5B). cette expérience démontre de façon univoque le rôle de ce site dans l'induction de la transcription d'IκBα par RORα1.

Compte tenu du fait que le gène RORα possède plusieurs isoformes (Giguère, V. et al. Genes & Development 8, 538-553 (1994), les inventeurs ont cherché à évaluer l'influence des isoformes RORα1, RORα2 et RORα3 sur des constructions sous le contrôle de deux copies de l'élément de réponse à RORα présent au niveau du promoteur du gène IκBα-RORE clonées en amont d'un promoteur hétérologue (promoteur TK).

RORα1 induit fortement l'activité du gène reporteur, ce qui démontre que IκBα-RORE peut fonctionner de façon indépendante du promoteur. Contrairement, RORα2 et RORα3 ne peuvent pas induire la transcription de la construction précédemment citée (figure 6A), ce qui indique que l'induction de la transcription de IκBα est spécifique à l'isoforme RORα1.

II a été montré que le dominant négatif RORαΔ235 auquel il manque le domaine LBD inhibe la transcription induite par RORα1 par compétition avec le même domaine de liaison à l'ADN (Mc Broom, L.D.B., Flock, G. & Giguère, V. Molecular Cellular Biology 15, 796-808, (1995)). L'effet de ce mutant sur le promoteur sous contrôle de RORE du gène IκBα induit par RORα1 a été testé. La co-transfection par RORαΔ235 abolit l'effet de RORα1 (figure 6B). Ceci démontre que la liaison de RORα au promoteur est essentielle à l'induction de la transcription. Enfin, la liaison de RORα1 à son élément de réponse RORE a été vérifiée par retard sur gel, en utilisant des protéines RORα1 traduites *in vitro*. Des expériences de compétition utilisant des sondes froides, natives ou mutées, ou des expériences de superdéplacement ont montré de façon univoque que RORα1 se liait fortement au site RORE présent dans le promoteur du gène IκBα.

Ainsi, lors des recherches effectuées dans le cadre de la présente invention, les inventeurs ont établi pour la première fois que la sur-expression de RORα1 dans les cellules musculaires lisses aortiques prévient l'expression de COX-2, de IL-6 et IL-8 induite par TNFα. Tous ces gènes sont des marqueurs majeurs de l'inflammation dans les lésions athérosclérotiques (Ross, R. New England Journal of Medicine 340, 115-126 (1999)). Les résultats issus de ces recherches montrent que RORα joue un rôle protecteur contre l'athérosclérose via un effet vasculaire direct en plus de son rôle modulateur du métabolisme des lipoprotéines (rôle qui passe par son action sur l'expression du gène de l'apolipoprotéine A1 (Mamontova, A. et al. Circulation 98, 2738-2743 (1998)). En régulant de façon négative l'IL-8, une chimiokine clé fortement impliquée dans le recrutement des monocytes en cours des phases initiales de l'athérosclérose (Ross, R. New England Journal of Medicine 340, 115-126 (1999)), RORα1 peut réduire l'initiation et la progression des lésions athérosclérotiques.

## Revendications

1. Procédé d'identification de substances utiles pour le traitement de l'inflammation, **caractérisé en ce qu'**il comprend les étapes suivantes :
a) la mise en contact de ladite substance avec une construction d'acide nucléique comprenant l'élément de réponse à ROR (Retinoid-related Orphan Receptor) du promoteur du gène IκBα, dans des conditions susceptibles de permettre à ladite substance de se fixer sur ledit élément de réponse,
b) la mesure de la fixation éventuelle de ladite substance sur l'élément de réponse ou de l'activité transcriptionnelle de l'élément de réponse ou d'un promoteur le contenant,
c) éventuellement la comparaison de la mesure de l'étape (b) avec une mesure réalisée dans les mêmes conditions qu'aux étapes (a) et (b) mais avec une construction d'acide nucléique comprenant l'élément de réponse muté.

2. Procédé d'identification de substances utiles pour le traitement de l'inflammation, **caractérisé en ce qu'**il comprend les étapes suivantes :
a) la mise en contact de ladite substance avec ROR (Retinoid-related Orphan Receptor) et une construction d'acide nucléique comprenant l'élément de réponse audit récepteur du promoteur du gène IκBα, dans des conditions susceptibles de permettre audit récepteur, à ladite substance ou au complexe formé du récepteur et de ladite substance de se fixer sur ledit élément de réponse,
b) la mesure de la fixation éventuelle de ROR, de la substance ou d'un complexe formé de ROR et de ladite substance sur l'élément de réponse ou de l'activité transcriptionnelle de l'élément de réponse ou d'un promoteur le contenant,
c) éventuellement la comparaison de la mesure de l'étape (b) avec une mesure réalisée dans les mêmes conditions qu'aux étapes (a) et (b) mais avec une construction d'acide nucléique comprenant l'élément de réponse muté.

3. Procédé selon l'une des revendications 1 ou 2, **caractérisé en ce qu'**à l'étape (a) la construction d'acide nucléique comprend au moins une copie de l'élément de réponse à ROR du promoteur du gène IκBα.

4. Procédé selon l'une quelconque des revendications précédentes, **caractérisé en ce que** l'élément de réponse est l'élément de réponse RORα et de préférence hRORα du promoteur du gène IκBα.

5. Procédé selon l'une quelconque des revendications précédentes, **caractérisé en ce que** la construction d'acide nucléique de l'étape (a) comprend tout ou partie du promoteur du gène IκBα contenant l'élément de réponse de ROR.

6. Procédé selon l'une quelconque des revendications précédentes, **caractérisé en ce que** la construction d'acide nucléique de l'étape (a) comprend ou est constituée par la séquence suivante :
GAGCACAATGTAGGTCAGATAG (SEQ ID No : 1)
ou un fragment de celle-ci.

7. Procédé selon l'une quelconque des revendications précédentes, **caractérisé en ce qu'**à l'étape (a), la construction d'acide nucléique comprend un gène rapporteur placé sous le contrôle du promoteur comprenant au moins un élément de réponse de ROR du gène IκBα.

8. Procédé selon l'une quelconque des revendications précédentes, **caractérisé en ce qu'**à l'étape (a), la construction d'acide nucléique comprend un gène rapporteur placé sous le contrôle du promoteur du gène IκBα.

9. Procédé selon l'une quelconque des revendications 7 ou 8, **caractérisé en ce que** le gène rapporteur est choisi parmi le gène IκBα, le gène de la chloramphénicol acétyltransférase, le gène de la luciférase de luciole ou de Renilla, le gène de la phosphatase alcaline secrétée ou le gène de la bêta-galactosidase.

10. Procédé d'identification de substances utiles pour le traitement de l'inflammation, **caractérisé en ce qu'**il comprend les étapes suivantes :
a) la mise en contact de ladite substance avec un hôte cellulaire transformée par une construction d'acide nucléique comprenant au moins un gène rapporteur combiné de manière fonctionnelle à l'élément de réponse à ROR (Retinoid-related Orphan Receptor) du promoteur du gène IκBα,
b) la mesure de l'expression du gène rapporteur par tout moyen approprié,
c) éventuellement la comparaison de la mesure de l'étape (b) avec une mesure réalisée dans les mêmes conditions qu'aux étapes (a) et (b) mais avec une construction d'acide nucléique comprenant l'élément de réponse muté.

11. Procédé selon la revendication 10, **caractérisé en ce que** l'hôte cellulaire de l'étape (a) exprime ROR.

12. Procédé selon l'une quelconque des revendications précédentes, **caractérisé en ce qu'**à l'étape (c), la comparaison de la mesure de l'étape (b) est avantageusement effectuée avec une mesure réalisée dans les mêmes conditions qu'aux étapes (a) et (b) mais avec une construction d'acide nucléique comprenant l'élément de réponse muté répondant à la séquence suivante : GAGCACAATGTXXXXXXGATAG (SEQ ID NO: 16), dans laquelle X est choisi parmi A, T, C, G.

## Claims

1. Method for identifying substances useful for treating inflammation, wherein said method comprises the following steps :
a) placing said substance in contact with a nucleic acid construct comprising the ROR (Retinoid-related Orphan Receptor) response element of the IκBα gene promoter, under conditions allowing said substance to bind to said response element,
b) measuring the possible binding of said substance to the response element or the transcriptional activity of the response element or of a promoter containing it,
c) optionally comparing the measurement of step (b) with a measurement carried out under the same conditions as those of steps (a) and (b) but with a nucleic acid construct containing the mutated response element.

2. Method for identifying substances useful for treating inflammation, wherein said method comprises the following steps :
a) placing said substance in contact with ROR (Retinoid-related Orphan Receptor) and a nucleic acid construct comprising the response element to said receptor of the IκBα gene promoter, under conditions allowing said receptor, said substance or the complex formed of the receptor and said substance, to bind to said response element,
b) measuring the possible binding of ROR, the substance or a complex formed of ROR and said substance to the response element or the transcriptional activity of the response element or of a promoter containing it,
c) optionally comparing the measurement of step (b) with a measurement carried out under the same conditions as those of steps (a) and (b) but with a nucleic acid construct containing the mutated response element.

3. Method according to any one of claims 1 and 2, wherein at step (a) the nucleic acid construct comprises at least one copy of the ROR response element of the IκBα gene promoter.

4. Method according to any one of the preceding claims, wherein the response element is the RORα, preferably hRORα, response element of the IκBα gene promoter.

5. Method according to any one of the preceding claims, wherein the nucleic acid construct of step (a) comprises all or part of the IκBα gene promoter containing the ROR response element.

6. Method according to any one of the preceding claims, wherein the nucleic acid construct of step (a) comprises or is constituted by the following sequence:
GAGCACAATGTAGGTCAGATAG (SEQ ID No : 1)
or a fragment thereof.

7. Method according to any one of the preceding claims, wherein at step (a), the nucleic acid construct comprises a reporter gene placed under the control of the promoter comprising at least one ROR response element of the IκBα gene.

8. Method according to any one of the preceding claims, wherein at step (a), the nucleic acid construct comprises a reporter gene placed under the control of the promoter of the IκBα gene.

9. Method according to any one of claims 7 and 8, wherein the reporter gene is selected from the IκBα gene, the chloramphenicol acetyltransferase gene, the firefly or Renilla luciferase gene, the secreted alkaline phosphatase gene and the beta-galactosidase gene.

10. Method for identifying substances useful for treating inflammation, wherein said method comprises the following steps :
a) placing said substance in contact with a host cell transformed with a nucleic acid construct comprising at least one reporter gene combined in a functional manner with the ROR (Retinoid-related Orphan Receptor) response element of the IκBα gene promoter,
b) measuring the expression of the reporter gene by any suitable means,
c) optionally comparing the measurement of step (b) with a measurement carried out under the same conditions as those of steps (a) and (b) but with a nucleic acid construct containing the mutated response element.

11. Method according to claim 10, wherein the host cell of step (a) expresses ROR.

12. Method according to any one of the preceding claims, wherein at step (c), the comparison of the measurement of step (b) is advantageously carried out with a measurement done under the same conditions as those of steps (a) and (b) but with a nucleic acid construct comprising the mutated response element having the following sequence : GAGCACAATGTXXXXXXGATAG, wherein X is selected from A, T, C, and G.

## Patentansprüche

1. Verfahren zur Identifizierung von Substanzen, die für die Behandlung von Entzündung zweckdienlich sind, **dadurch gekennzeichnet, dass** es die folgenden Schritte umfasst:
a) das Inkontaktbringen besagter Substanz mit einem Nukleinsäure-Konstrukt, umfassend das Antwortelement auf ROR (Retinoid-related Orphan Receptor) des Promotors des IκBα-Gens, unter geeigneten Bedingungen, die besagter Substanz erlauben, an besagtes Antwortelement zu binden,
b) die Messung der möglichen Bindung besagter Substanz an das Antwortelement oder der Transkriptionsaktivität des Antwortelements oder eines das Antwortelement enthaltenden Promotors,
c) gegebenenfalls den Vergleich der Messung von Schritt (b) mit einer Messung, die unter denselben Bedingungen wie bei den Schritten (a) und (b) aber mit einem Nukleinsäure-Konstrukt durchgeführt wird, welches das mutierte Antwortelement umfasst.

2. Verfahren zur Identifizierung von Substanzen, die für die Behandlung von Entzündung zweckdienlich sind, **dadurch gekennzeichnet, dass** es die folgenden Schritte umfasst:
a) das Inkontaktbringen besagter Substanz mit ROR (Retinoid-related Orphan Receptor) und einem Nukleinsäure-Konstrukt, umfassend das Antwortelement auf besagten Rezeptor des Promotors des IκBα-Gens, unter geeigneten Bedingungen, die besagtem Rezeptor, besagter Substanz oder dem von dem Rezeptor und besagter Substanz gebildeten Komplex erlauben, an besagtes Antwortelement zu binden,
b) die Messung der möglichen Bindung von ROR, der Substanz oder eines von ROR und besagter Substanz gebildeten Komplexes an das Antwortelement oder der Transkriptionsaktivität des Antwortelements oder eines das Antwortelement enthaltenden Promotors,
c) gegebenenfalls den Vergleich der Messung von Schritt (b) mit einer Messung, die unter denselben Bedingungen wie bei den Schritten (a) und (b) aber mit einem Nukleinsäure-Konstrukt durchgeführt wird, welches das mutierte Antwortelement umfasst.

3. Verfahren gemäß einem der Ansprüche 1 oder 2, **dadurch gekennzeichnet, dass** bei Schritt (a) das Nukleinsäure-Konstrukt wenigstens eine Kopie des Antwortelements auf ROR des Promotors des IκBα-Gens umfasst.

4. Verfahren gemäß einem der vorhergehenden Ansprüche, **dadurch gekennzeichnet, dass** das Antwortelement das RORα-Antwortelement und vorzugsweise hRORα des Promotors des IκBα-Gens ist.

5. Verfahren gemäß einem der vorhergehenden Ansprüche, **dadurch gekennzeichnet, dass** das Nukleinsäure-Konstrukt von Schritt (a) den gesamten oder einen Teil des Promotors des IκBα-Gens umfasst, der das ROR-Antwortelement enthält.

6. Verfahren gemäß einem der vorhergehenden Ansprüche, **dadurch gekennzeichnet, dass** das Nukleinsäure-Konstrukt von Schritt (a) die folgende Sequenz:
GAGCACAATGTAGGTCAGATAG (SEQ ID NR.: 1)
oder ein Fragment davon umfasst oder daraus besteht.

7. Verfahren gemäß einem der vorhergehenden Ansprüche, **dadurch gekennzeichnet, dass** bei Schritt (a) das Nukleinsäure-Konstrukt ein Reportergen unter der Kontrolle des Promotors umfasst, der wenigstens ein ROR-Antwortelement des IκBα-Gens umfasst.

8. Verfahren gemäß einem der vorhergehenden Ansprüche, **dadurch gekennzeichnet, dass** bei Schritt (a) das Nukleinsäure-Konstrukt ein Reportergen unter der Kontrolle des Promotors des IκBα-Gens umfasst.

9. Verfahren gemäß einem der Ansprüche 7 oder 8, **dadurch gekennzeichnet, dass** das Reportergen aus dem IκBα-Gen, dem Chloramphenicol-Acetyltransferase-Gen, dem Luciferase-Gen der Leuchtkäfer oder der Renilla, dem Gen der sezernierten alkalischen Phosphatase oder dem beta-Galactosidase-Gen ausgewählt ist.

10. Verfahren zur Identifizierung von Substanzen, die für die Behandlung von Entzündung zweckdienlich sind, **dadurch gekennzeichnet, dass** es die folgenden Schritte umfasst:
a) das Inkontaktbringen besagter Substanz mit einer Wirtszelle, die mit einem Nukleinsäure-Konstrukt transformiert ist, das wenigstens ein mit dem Antwortelement auf ROR (Retinoid-related Orphan Receptor) des Promotors des IκBα-Gens funktionsfähig verbundenes Reportergen umfasst,
b) die Messung der Expression des Reportergens mit einem beliebigen geeigneten Mittel,
c) gegebenenfalls den Vergleich der Messung von Schritt (b) mit einer Messung, die unter denselben Bedingungen wie bei den Schritten (a) und (b) aber mit einem Nukleinsäure-Konstrukt durchgeführt wird, welches das mutierte Antwortelement umfasst.

11. Verfahren gemäß Anspruch 10, **dadurch gekennzeichnet, dass** die Wirtszelle von Schritt (a) ROR exprimiert.

12. Verfahren gemäß einem der vorhergehenden Ansprüche, **dadurch gekennzeichnet, dass** bei Schritt (c) der Vergleich der Messung von Schritt (b) vorteilhafterweise mit einer Messung bewerkstelligt wird, die unter denselben Bedingungen wie bei den Schritten (a) und (b) aber mit einem Nukleinsäure-Konstrukt durchgeführt wird, welches das mutierte Antwortelement umfasst, das auf die folgende Sequenz: GAGCACAATGTXXXXXXGATAG (SEQ ID NR.: 16) antwortet, in der X aus A, T, C, G ausgewählt ist.


### LISTE DE SEQUENCES

<110> GENFIT SA
<120> Procédé d'identification de substances utiles pour le traitement de l'inflammation mettant en oeuvre l'élément de réponse au récepteur ROR du gène IkBa.
<130> B0079WO
<140>
   <141>
<160> 15
<170> PatentIn Ver. 2.1
<210> 1
   <211> 22
   <212> ADN
   <213> Homo sapiens
<400> 1
   gagcacaatg taggtcagat ag 22
<210> 2
   <211> 21
   <212> ADN
   <213> Séquence artificielle
<220>
   <223> Description de la séquence artificielle: amorce
<400> 2
   gtcagcagct tctacctgga c 21
<210> 3
   <211> 26
   <212> ADN
   <213> Séquence artificielle
<220>
   <223> Description de la séquence artificielle: amorce
<400> 3
   gtgttgttct gagagtgaaa ggcacg 26
<210> 4
   <211> 30
   <212> ADN
   <213> Séquence artificielle
<220>
   <223> Description de la séquence artificielle: amorce
<400> 4
   atgcagcccc gaatgctcct catcgtggcc 30
<210> 5
   <211> 23
   <212> ADN
   <213> Séquence artificielle
<220>
   <223> Description de la séquence artificielle: amorce
<400> 5
   ttcttggagg ccatgtgggc cat 23
<210> 6
   <211> 8
   <212> ADN
   <213> Séquence artificielle
<220>
   <223> Description de la séquence artificielle: Site RORE
<400> 6
   taggtcag 8
<210> 7
   <211> 10
   <212> ADN
   <213> Séquence artificielle
<220>
   <223> Description de la séquence artificielle: Site RORE
<400> 7
   gtaggtcaga 10
<210> 8
   <211> 14
   <212> ADN
   <213> Séquence artificielle
<220>
   <223> Description de la séquence artificielle: Site RORE
<400> 8
   atgtaggtca gata 14
<210> 9
   <211> 22
   <212> ADN
   <213> Séquence artificielle
<220>
   <223> Description de la séquence artificielle: site RORE muté
<400> 9
   gagcacaatg tatttcagat ag 22
<210> 10
   <211> 22
   <212> ADN
   <213> Séquence artificielle
<220>
   <223> Description de la séquence artificielle: site RORE muté
<400> 10
   gagcacaatg taaatcagat ag 22
<210> 11
   <211> 22
   <212> ADN
   <213> Séquence artificielle
<220>
   <223> Description de la séquence artificielle: site RORE muté
<400> 11
   gagcacaatg tacatcagat ag 22
<210> 12
   <211> 22
   <212> ADN
   <213> Séquence artificielle
<220>
   <223> Description de la séquence artificielle: site RORE muté
<400> 12
   gagcacaatg taactcagat ag 22
<210> 13
   <211> 22
   <212> ADN
   <213> Séquence artificielle
<220>
   <223> Description de la séquence artificielle: site RORE muté
<400> 13
   gagcacaatg tatatcagat ag 22
<210> 14
   <211> 22
   <212> ADN
   <213> Séquence artificielle
<220>
   <223> Description de la séquence artificielle: site RORE muté
<400> 14
   gagcacaatg taattcagat ag 22
<210> 15
   <211> 22
   <212> ADN
   <213> Séquence artificielle
<220>
   <223> Description de la séquence artificielle: site RORE muté
<400> 15
   gagcacaatg tacctcagat ag 22